# EUROPEAN PATENT APPLICATION

(11) **EP 3 300 716 A1**
(43) Date of publication of application: **04.04.2018**
(21) Application number: 16768176.6
(22) Date of filing: 04.02.2016
(51) Int. Cl.: A61K 8/37, A61K 31/23, A61P 1/02, A61Q 11/00

(54) **BIOFILM FORMATION INHIBITOR**

(30) Priority: 23.03.2015 JP 2015059262
(71) Applicant: Riken Vitamin Co., Ltd., Chiyoda-ku, Tokyo 101-8370 (JP)
(72) Inventor: KOIDE, Tomojiro, Chiba-shi Chiba 261-0002 (JP)
(74) Representative: Witte, Weller & Partner Patentanwälte mbB
(86) International application number: PCT/JP2016/053415
(87) International publication number: WO 2016/152273

(57) **Abstract**

A biofilm formation inhibitor comprising monoglyceryl dicaprylate and/or diglyceryl dicaprylate as an active ingredient is useful as a biofilm formation inhibitor capable of inhibiting biofilm formation without killing microorganisms.

## Description

### TECHNICAL FIELD

The present invention relates to a biofilm formation inhibitor used for inhibition of microbial biofilm formation.

### BACKGROUND ART

A biofilm is an aggregate of microorganisms such as bacteria and fungi adherent to a solid or liquid surface, and exists in various environments. This microbial aggregate called biofilm is closely involved in the fields of daily life, food industry, medical care, and the like, and has caused many problems. For example, in the field of daily life, slime in drain outlets in a kitchen, a bathroom, a toilet, and the like is also a biofilm, and is pointed out as a possible cause of odor. In the field of food industry, microorganisms may adhere to pipes, production equipment etc. and form a biofilm, which is pointed out as a possible cause of microbial contamination. In the field of medical care, a biofilm may be formed inside catheters and on contact lenses, dentures, etc., and is pointed out as a possible cause of infections.

Particularly on dentures, dirt causes denture stomatitis as well as angular stomatitis etc., which may lead to promotion of biofilm formation (denture plaque deposition) and thus further worsening of oral cavity environment. In such a biofilm, *Candida* species are reported to be frequently detected.

Generally, biofilms, once formed, acquire resistance to chemical permeation and heat, and therefore are difficult to remove by treatment with chemicals or heat. Physical removal by brushing etc. is considered as the most effective, but even such a physical method still has the disadvantage of difficulty in meticulous removal of biofilms. Other methods are proposed for biofilm removal, including a method using a common microbicide, a method using a substance for biofilm dissolution, and a method using a combination of both of them. However, frequent or high-dose use of a microbicide may have various influences on the human body, including the emergence of drug resistant microorganisms, the onset of microbial substitution due to microbicide-induced changes in oral cavity flora, the manifestation of side effects, etc. Therefore, instead of killing biofilm-forming microorganisms, inhibiting biofilm formation is an effective way.

Known techniques for inhibiting biofilm formation include a biofilm inhibitor for inhibiting *Candida* biofilm formation which inhibitor comprises a monoglycerin mono-fatty acid ester having a saturated fatty acid residue of 7 to 14 carbon atoms (including monoglyceryl monocaprylate) and diglyceryl monolaurate as active ingredients (Patent Literature 1). However, as is seen in Table 2 of Patent Literature 1, glyceryl dilaurate (monoglycerin di-fatty acid ester) is less effective against biofilm formation than glyceryl monolaurate (monoglycerin mono-fatty acid ester), that is, a monoglycerin fatty acid ester molecule containing two fatty acid molecules is unfavorable. Further, as is also seen in Table 2 of Patent Literature 1, diglyceryl monocaprylate (diglycerin mono-caprylic acid ester) is poorly effective against biofilm formation, that is, caprylic acid is unfavorable as a fatty acid moiety of a diglycerin fatty acid ester.

### CITATION LIST

### Patent Literature

Patent Literature 1: JP-A 2013-231002

### SUMMARY OF INVENTION

### TECHNICAL PROBLEM

An object of the present invention is to provide a biofilm formation inhibitor capable of inhibiting biofilm formation without killing microorganisms.

### SOLUTION TO PROBLEM

The present inventor conducted extensive research to achieve the above-mentioned object, and as a result, found that monoglyceryl dicaprylate and/or diglyceryl dicaprylate can achieve the above-mentioned object. Based on the findings, the present inventor conducted further research and completed the present invention. That is, the present invention includes the following.
[1] A biofilm formation inhibitor comprising monoglyceryl dicaprylate and/or diglyceryl dicaprylate as an active ingredient.
[2] An oral composition for inhibiting biofilm formation, comprising the biofilm formation inhibitor according to the above [1].
[3] A method for inhibiting biofilm formation, comprising using monoglyceryl dicaprylate and/or diglyceryl dicaprylate.
[4] A method for inhibiting biofilm formation, comprising bringing microorganisms into contact with monoglyceryl dicaprylate and/or diglyceryl dicaprylate.
[5] Monoglyceryl dicaprylate and/or diglyceryl dicaprylate for use in inhibition of biofilm formation.

### ADVANTAGEOUS EFFECTS OF INVENTION

The biofilm formation inhibitor of the present invention can inhibit the formation of *Candida* biofilm without killing *Candida* species.

### DESCRIPTION OF EMBODIMENTS

The monoglyceryl dicaprylate, which is an active ingredient of the biofilm formation inhibitor of the present invention, is composed of one monoglycerin molecule bound to two caprylic acid molecules via ester bonds.

The monoglyceryl dicaprylate may be in the form of a mixture containing monoglyceryl dicaprylate, for example, a mixture of esters of monoglycerin and caprylic acid, or a mixture of products obtained by transesterification of monoglycerin or monoglyceryl monocaprylate with monoglyceryl tricaprylate. These mixtures may usually contain, in addition to monoglyceryl dicaprylate, monoglyceryl monocaprylate, monoglyceryl tricaprylate, monoglycerin, caprylic acid, etc.

In the mixture containing monoglyceryl dicaprylate, the diester content in the esters is preferably 40% by weight or more, and more preferably 70% by weight or more.

The production method of the monoglyceryl dicaprylate is not particularly limited and may be a known method or a method known per se. Generally, the monoglyceryl dicaprylate can be produced by esterification of monoglycerin and caprylic acid.

The caprylic acid used as a source material of the monoglyceryl dicaprylate is not particularly limited as long as it is from edible fats and oils of animal or vegetable origin, but preferably has a purity of 50% or more. When the purity is less than 50%, the biofilm formation inhibitory effect of the present invention may not be sufficiently produced. The caprylic acid may be obtained by chemical synthesis.

The production method of the monoglyceryl dicaprylate using esterification is, for example, as follows. Into a common reaction container equipped with a mixer, a heating jacket, a baffle plate, etc., monoglycerin and caprylic acid are fed at a molar ratio of 1.0:0.75 to 1.0:2.1, and usually, an alkali (e.g., sodium hydroxide etc.) is added as a catalyst. The mixture is stirred and heated at a predetermined temperature under a nitrogen gas atmosphere to allow esterification to proceed while water generated by the reaction is removed from the system. The reaction temperature is usually in the range of about 180 to 260°C, and preferably about 200 to 250°C. The pressure condition of the reaction is normal pressure or reduced pressure, and the reaction time is about 0.5 to 15 hours, and preferably about 1 to 3 hours. To determine the end point of the reaction, the acid value of the reaction mixture is usually measured. When the the acid value is about 3 or less, the reaction is regarded as completed. The resulting reaction mixture is a mixture containing unreacted caprylic acid, unreacted monoglycerin, monoglyceryl monocaprylate, monoglyceryl dicaprylate, monoglyceryl tricaprylate, etc. This mixture may be directly used in the present invention.

After the completion of the esterification, the catalyst remaining in the reaction mixture may be neutralized if desired. When the temperature of the esterification is 200°C or more, the temperature of the reaction mixture is preferably cooled down to 180 to 200°C before neutralization. After the neutralization, in a preferable embodiment, unreacted monoglycerin and triglyceride are removed as much as possible from the reaction mixture, and thereby monoglyceryl dicaprylate is obtained. For the removal of unreacted glycerin and triglyceride, methods known per se can be used, including, for example, vacuum distillation, molecular distillation, column chromatography, liquid-liquid extraction, and the like. After the removal of unreacted glycerin and triglyceride, decolorization, deodorization and other treatments can be performed if desired.

As the monoglyceryl dicaprylate, commercially available products of monoglyceryl dicaprylate can also be used. Examples include Sunfat GDC-S (trade name; manufactured by Taiyo Kagaku Co., Ltd.; diester content: 75%).

The diglyceryl dicaprylate, which is another active ingredient of the biofilm formation inhibitor of the present invention, is composed of one diglycerin molecule bound to two caprylic acid molecules via ester bonds.

The diglyceryl dicaprylate may be in the form of a mixture containing diglyceryl dicaprylate, for example, a mixture of esters of diglycerin and caprylic acid. This mixture may usually contain, in addition to diglyceryl dicaprylate, diglyceryl monocaprylate, diglyceryl tricaprylate, diglyceryl tetracaprylate, diglycerin, caprylic acid, etc.

In the mixture containing diglyceryl dicaprylate, the diester content in the esters is preferably 20% or more, and more preferably 35% or more.

The diglycerin used as a source material of the biofilm formation inhibitor of the present invention is, for example, a diglycerin mixture having an average polymerization degree of about 1.5 to 2.4, preferably about 2.0. Such a diglycerin mixture can usually be obtained by polycondensation of glycerin, preferably in the presence of a small amount of an acid or an alkali as a catalyst. The polycondensation is performed under heating at a temperature of, for example, about 180°C or more, in the atmosphere of an inert gas such as nitrogen or carbon dioxide. Alternatively, the diglycerin may be obtained from glycidol or epichlorohydrin as a source material. After the completion of the reaction, neutralization, desalination, decolorization and other treatments may be performed if needed.

In the present invention, preferably, the above diglycerin mixture is purified by a known method or a method known per se such as distillation and column chromatography, and the resulting high-purity diglycerin is used. The high-purity diglycerin contains about 50% by mass or more, preferably 85% by mass or more of diglycerin consisting of two glycerin molecules.

The caprylic acid used as a source material of the diglyceryl dicaprylate is not particularly limited as long as it is from edible fats and oils of animal or vegetable origin, but preferably has a purity of 50% or more. When the purity is less than 50%, the biofilm formation inhibitory effect of the present invention may not be sufficiently produced. The caprylic acid may be obtained by chemical synthesis.

The diglyceryl dicaprylate as an active ingredient of the biofilm formation inhibitor of the present invention can be produced by esterification as exemplified in the following. Into a common reaction container equipped with a mixer, a heating jacket, a baffle plate, etc., diglycerin and caprylic acid are fed at a molar ratio of about 1.0:0.7 to about 1.0:2.5, and usually, sodium hydroxide is added as a catalyst. The mixture is stirred and heated at a predetermined temperature under a nitrogen gas atmosphere to allow esterification to proceed while water generated by the reaction is removed from the system. The predetermined temperature is usually in the range of about 180 to 260°C, and preferably about 200 to 250°C. The pressure condition of the reaction is reduced pressure or normal pressure, and the reaction time is about 0.5 to 15 hours, and preferably about 1 to 3 hours. To determine the end point of the reaction, the acid value of the reaction mixture is usually measured. Preferably, when the the acid value is about 3 or less, the reaction is regarded as completed. The resulting reaction mixture is a mixture containing unreacted caprylic acid, unreacted diglycerin, diglyceryl monocaprylate, diglyceryl dicaprylate, diglyceryl tricaprylate, diglyceryl tetracaprylate, etc. This mixture may be directly used in the present invention.

After the completion of the esterification, the catalyst remaining in the reaction mixture may be neutralized if desired. When the temperature of the esterification is about 200°C or more, the temperature of the reaction mixture is preferably cooled down to about 180 to 200°C before neutralization. After the neutralization, the reaction mixture is cooled if desired, and allowed to stand at a constant temperature of about 100 to 180°C, preferably about 130 to 150°C, for a certain period, which is preferably about 0.5 hour or more, and more preferably about 1 to 10 hours. If unreacted diglycerin has sunk to the bottom, it is preferably removed.

In the diglyceryl dicaprylate obtained after the above treatment, the diester content is usually about 20% or more and less than 40%. If desired, such a diglyceryl dicaprylate may be purified by a method known per se, so that the diester content can be about 50% or more, preferably about 70% or more relative to the whole. The method known per se is, for example, molecular distillation using a falling-film molecular distiller, a centrifugal molecular distiller or other types of distillers, column chromatography, liquid-liquid extraction, or the like. After the distillation, decolorization, deodorization and other treatments may be performed if desired.

As the diglyceryl dicaprylate, commercially available products of diglyceryl dicaprylate can be used. Examples include POEM FB-28 (trade name; manufactured by Riken Vitamin Co., Ltd.; diester content: 38%).

The biofilm formation inhibitor of the present invention is effective for the inhibition of biofilm formation by harmful microorganisms, particularly *Candida* species. The *Candida* species are not particularly limited and refer to all fungi belonging to the genus *Candida.* Specific examples include *Candida albicans, Candida tropicalis, Candida parapsilosis, Candida glabrata, Candida guilliermondii* and *Candida krusei.*

An oral composition for inhibiting biofilm formation which composition comprises a biofilm formation inhibitor is also one embodiment of the present invention.

The oral composition for inhibiting biofilm formation comprises monoglyceryl dicaprylate and/or diglyceryl dicaprylate, each of which is the biofilm formation inhibitor of the present invention, and is in the form of, for example, a dentifrice which may be paste, liquid, foam, or the like, a denture stabilizer, a gingival massage cream, a topical liniment, a mouth rinse, a mouthwash, a mouth deodorant, a tablet, a wet wipe for oral use, or a moisturizing gel for oral use.

A food product for inhibiting biofilm formation which product comprises a biofilm formation inhibitor is also one embodiment of the present invention. The food product for inhibiting biofilm formation comprises monoglyceryl dicaprylate and/or diglyceryl dicaprylate, each of which is the biofilm formation inhibitor of the present invention, and is in the form of, for example, a troche, a chewing gum or a film-shaped food product. The food product for inhibiting biofilm formation can prevent denture plaque deposition and the like.

In addition, the embodiments of the present invention include a cosmetic product, a quasi-pharmaceutical product, a pharmaceutical product and other products for inhibiting biofilm formation each of which products comprises a biofilm formation inhibitor.

In the above-mentioned oral composition, food product, cosmetic product, quasi-pharmaceutical product, pharmaceutical product and other products for inhibiting biofilm formation, the amount of the biofilm formation inhibitor is preferably 0.0001 to 5.0% by mass, and more preferably 0.0005 to 1.0% by mass in terms of the active ingredient(s), that is, monoglyceryl dicaprylate and/or diglyceryl dicaprylate. When the amount is less than 0.0001% by mass, the biofilm formation inhibitory effect may be insufficient. When the amount is more than 5.0% by mass, adverse effects on the taste may occur.

Hereinafter, the present invention will be illustrated by examples. The following examples are merely descriptive of the present invention and are not intended to limit the present invention in any way.

### EXAMPLES

### Present Invention Products and Reference Products

### Present Invention Products

The present invention products used were the present invention products 1, 2, 3 and 4 shown below.
Present invention product 1: monoglyceryl dicaprylate (Trade name: Sunfat GDC-S; manufactured by Taiyo Kagaku Co., Ltd.)
Present invention product 2: diglyceryl dicaprylate (prepared by the method described later)
Present invention product 3: diglyceryl dicaprylate (prepared by the method described later)
Present invention product 4: a mixture of the present invention products 1 and 2 (mass ratio of the present invention products 1 and 2 = 1:1)

### Reference Products

The reference products used were the reference products 1 and 2 shown below.
Reference product 1: monoglyceryl dilaurate
   (Trade name: α,α'-dilaurin (reagent); manufactured by Tokyo Chemical Industry Co., Ltd., purity: 96% or more)
Reference product 2: ketoconazole
   (Trade name: Ketoconazole; manufactured by LKT Laboratories Inc.)

### Production of Present Invention Product 2

Twenty kilograms of glycerin was fed into a reaction vessel equipped with a stirrer, a thermometer, a gas blowing pipe and a water separator. To this, 100 mL of a 20 w/v% aqueous sodium hydroxide solution was added as a catalyst, and the mixture was kept in nitrogen gas flow at 250°C for 4 hours to allow glycerol condensation to proceed. The obtained reaction product was cooled to about 90°C, and neutralization with about 20 g of phosphoric acid was performed, followed by filtration. The filtrate was distilled under reduced pressure of 250 Pa at 160°C for removal of glycerin. Subsequently, vacuum distillation was performed under high vacuum of 20 Pa at 200°C to give an about 3.0-kg fraction (diglycerin-containing mixture) containing 3% glycerin, 92% diglycerin and 5% triglycerin. The fraction was treated with 1% by mass of activated carbon under reduced pressure for decolorization, and then filtered. The obtained diglycerin had a hydroxyl value of about 1359, an average polymerization degree of about 2.0, and a diglycerin content of 98% by mass.

Next, 176.6 g (about 1.06 mol) of the diglycerin obtained by the above procedure and 123.4 g (about 0.86 mol) of caprylic acid (trade name: NAA-82; manufactured by NOF CORPORATION) were fed into a 500-mL four-neck flask equipped with a mixer, a thermometer, a gas blowing pipe and a water separator. To this, 1.5 mL of a 10 w/v% aqueous sodium hydroxide solution was added as a catalyst, and the mixture was kept in nitrogen gas flow at 200°C under normal pressure for 1 hour to allow esterification to proceed. The temperature was raised to 230°C, and the esterification was further continued for about 2 hours until the acid value became 1.0 or less. The obtained reaction mixture was cooled, and thus about 270 g of the present invention product 2 was obtained.

### Production of Present Invention Product 3

To the present invention product 2 (100 g) obtained by the above procedure, 100 g of hexane was added, and the whole was mixed in a separatory funnel. To this, 50 g of 20% by mass hydrous methanol was added, and the whole was mixed. The lower phase (aqueous phase) was separated and designated as aqueous phase fraction 1. Subsequently, 50 g of 20% by mass hydrous methanol was added to the remaining upper phase, and the whole was mixed. The lower phase was separated and designated as aqueous phase fraction 2. This procedure was repeated 5 more times, and thereby aqueous phase fractions 3 to 7 were obtained. The aqueous phase fractions 4 to 7 were combined. To the combined aqueous phase fractions 4 to 7 (230 g in total), 70 g of hexane was added, and the whole was mixed. After separation of the lower phase, the solvent in the lower phase was evaporated off in vacuo with a rotary evaporator, and thus about 14 g of the present invention product 3 was obtained.

The amounts of different types of esters, that is, monoester, diester and triester in each of the present invention products 1, 2 and 3 are summarized in Table 1. The amounts of different types of esters were measured according to the following method.

### Method for Measuring Amounts of Different Types of Esters

The composition of the esters was analyzed by HPLC, and for quantitative determination, an absolute calibration method was used. That is, with use of a data processor, HPLC peak areas corresponding to the different types of esters on the recorded chromatogram of each sample were measured, and the amounts of these esters were calculated from the respective calibration curves produced using the corresponding standard samples, which were monoglyceryl monostearate, diglyceryl monostearate and triglyceryl monostearate each purified by normal phase column chromatography. The HPLC analysis conditions are shown below.

### HPLC Analysis Conditions

| | |
|---|---|
| System | Shimadzu high-performance liquid chromatography |
| Pump | (model: LC-10A; manufactured by Shimadzu Corporation) |
| Column oven | (model: CTO-10A; manufactured by Shimadzu Corporation) |
| Data processor | (model: C-R7A; manufactured by Shimadzu Corporation) |
| Column | GPC column (model: SHODEX KF-802; manufactured by Showa Denko K.K.) two columns connected in series |
| Mobile phase | THF |
| Flow rate | 1.0 mL/min |
| Detector | RI detector (model: RID-6A; manufactured by Shimadzu Corporation) |
| Column temperature | 40°C |
| Sample injection volume | 15 µL (in THF) |

**[Table 1]**

| | Type | Distributor etc. | Amounts of different types of esters (%) | | |
|---|---|---|---|---|---|
| | | | monoester | diester | triester |
| Present invention product 1 | Monoglyceryl dicaprylate | Taiyo Kagaku Co., Ltd. | 3 | 75 | 20 |
| Present invention product 2 | Diglyceryl dicaprylate | - | 20 | 38 | 30 |
| Present invention product 3 | Diglyceryl dicaprylate | - | 14 | 78 | 7 |

### Evaluation of Biofilm Formation Inhibitory Effect

The present invention products 1, 2, 3 and 4 and the reference products 1 and 2 were evaluated for inhibitory effect on biofilm formation by a test fungus (*Candida albicans* NBRC 1594).

### (1) Preparation of Test Fungal Suspension

A potato dextrose agar (manufactured by Difco) was sterilized by autoclaving at 121°C for 15 minutes, added to a sterilized culture dish, and solidified to prepare an agar plate. A cryopreserved test fungus was spread on this agar plate and cultured at 30°C for 2 days. A yeast nitrogen base with 2% glucose (manufactured by Difco) was diluted 10-fold and sterilized by autoclaving at 121°C for 15 minutes. Twenty milliliters of this sterilized medium was placed into a conical flask with a baffle, and one loop of the agar-cultured test fungus was inoculated into the conical flask. Preculture was performed at 30°C with agitation at 150 rpm for 24 hours. Then, the fungal cells were collected by centrifugation (4700 rpm, 10 minutes, 20°C) . The collected fungal cells were washed with 20 mL of physiological saline and collected by centrifugation again (4700 rpm, 10 minutes, 20°C) . The collected fungal cells were resuspended in 10 mL of a Sabouraud-dextrose broth (liquid medium which contained 1.0% by mass of peptone and 4.0% by mass of glucose and was adjusted to a pH of 5.7 with 1 N hydrochloric acid, and hereinafter, it is called "SG medium"). This resuspension was diluted 20-fold with SG medium and used as a test fungal suspension. The number of fungal cells in the test fungal suspension was 3.2 × 10⁶ cells/mL.

### (2) Protocol for Biofilm Formation

The present invention products 1, 2, 3 and 4 and the reference products 1 and 2 were used as test samples. The test samples were separately dissolved in dimethyl sulfoxide (DMSO) to prepare test sample solutions. Two microliters of each test sample solution and 198 µL of the test fungal suspension were added to wells of a 96-well microplate (trade name: TC plate 96 well (Cell+) ; manufactured by Sarstedt) . The concentration of the test sample in the test sample solution was such that the concentration of the test sample in the combined total amount of the test sample solution and the test fungal suspension, i.e., the concentration of the test sample in the well would be 5 ppm, 25 ppm, 50 ppm or 100 ppm. Static culture was performed in the 96-well microplate containing the test sample solution and the test fungal suspension at 30°C for 2 days to allow biofilm formation to proceed in each well.

As a control (without any test sample), 2 µL of DMSO alone and 198 µL of the test fungal suspension were added to other wells instead of 2 µL of the test sample solution and 198 µL of the test fungal suspension, and except for this, the same procedure was performed to allow biofilm formation to proceed in each well.

### (3) Protocol for Quantification of Biofilm Formation

After the static culture, the liquid in each well, including the test sample solution, the culture medium and the like, was removed, and the residue adherent to each well (cells, secretions, deposits, etc.) was regarded as a biofilm. To each well, 50 µL of a 0.01% by mass aqueous crystal violet solution (trade name, manufactured by Becton, Dickinson and Company) was added for staining of the biofilm. Subsequently, the crystal violet solution was removed from each well, and each well was washed twice with 200 µL of distilled water. Then, 200 µL of ethanol was added to each well to elute the crystal violet bound to the biofilm, and the degree of formation of the biofilm adherent to the microplate was quantified. The quantification was performed by measuring the absorbance at 600 nm (O.D. 600) in each well with a microplate reader (model: SH-9000 Lab; manufactured by CORONA ELECTRIC Co., Ltd.).

### (4) Protocol for Evaluation of Biofilm Formation Inhibitory Effect

The absorbance at 600 nm measured for the biofilm formed in the control well was used as the reference absorbance and assumed as 100% in terms of the degree of biofilm formation. The degree of biofilm formation for each test sample was calculated by comparison of the absorbance for the test sample with the reference absorbance. The degree of biofilm formation for each test sample was plotted on the vertical axis and the concentration of each test sample was plotted on the horizontal axis, thereby producing a graph of a logarithmic approximation curve.

The graph of the logarithmic approximation curve was used to calculate the concentration of the test sample where the absorbance for the test sample is equal to 50% of the reference absorbance, that is, the degree of biofilm formation was 50% (hereinafter called "50% inhibitory concentration").

For the evaluation of each test sample for the biofilm formation inhibitory effect, the 50% inhibitory concentration of each test sample was compared with the 50% inhibitory concentration of the reference product 2 (ketoconazole as a positive control). When the 50% inhibitory concentration of the test sample was 20 ppm or less, the test sample was regarded as excellent in the inhibition of biofilm formation. In contrast, when the 50% inhibitory concentration of the test sample was more than 20 ppm, the test sample was regarded as poor in the inhibition of biofilm formation. The results are shown in Table 2.

**[Table 2]**

| Test sample | 50% inhibitory concentration | Evaluation |
|---|---|---|
| Present invention product 1 | 3.9 ppm | Excellent in inhibition of biofilm formation |
| Present invention product 2 | 8.7 ppm | Excellent in inhibition of biofilm formation |
| Present invention product 3 | 3.2 ppm | Excellent in inhibition of biofilm formation |
| Present invention product 4 | 5.5 ppm | Excellent in inhibition of biofilm formation |
| Reference product 1 | 100 ppm or more | Poor in inhibition of biofilm formation |
| Reference product 2 | 23.8 ppm | Poor in inhibition of biofilm formation |

### Evaluation of Fungistatic Effect of Test Samples Used for Inhibition of Biofilm Formation

Ten milliliters of a brain heart infusion agar (manufactured by Difco) was sterilized by autoclaving at 121°C for 15 minutes and then cooled down to 50°C. To this, 100 µL of a solution of the test sample in dimethyl sulfoxide (DMSO) was added, and the mixture was stirred until homogeneous. The homogeneous mixture was added to a sterilized culture dish to prepare an agar plate. The concentration of the test sample in the agar plate was 10 ppm, 100 ppm, 300 ppm or 1000 ppm. Next, one loop of a cryopreserved test fungus (*Candida albicans* NBRC 1594) was streaked on each agar plate and cultured at 30°C for 1 day.

After the 1-day culture, the fungal growth state on each agar plate was visually observed. When no growth was observed along the streak line on the agar plate, the test sample was determined to be fungistatic at the indicated concentration. The results are shown in Table 3.

**[Table 3]**

| Test sample | Evaluation of fungistatic effect |
|---|---|
| Present invention product 1 | Fungal growth was observed in a 1000 ppm-treated plot. No fungistatic effect. |
| Present invention product 2 | Fungal growth was observed in a 1000 ppm-treated plot. No fungistatic effect. |
| Present invention product 3 | Fungal growth was observed in a 1000 ppm-treated plot. No fungistatic effect. |
| Present invention product 4 | Fungal growth was observed in a 1000 ppm-treated plot. No fungistatic effect. |
| Reference product 1 | Fungal growth was observed in a 1000 ppm-treated plot. No fungistatic effect. |
| Reference product 2 | No fungal growth was observed in a 100 ppm-treated plot. Fungistatically effective. |

The results in Tables 2 and 3 show the following findings. As for the evaluation of the present invention products 1, 2, 3 and 4 for biofilm formation inhibitory effect, each of them had a 50% inhibitory concentration below 20 ppm and was excellent in the biofilm formation inhibitory effect. As for the fungistatic evaluation, fungal growth was observed in a plot treated with any of the present invention products even at 1000 ppm, and none of them were fungistatic. That is, none of the present invention products 1, 2, 3 and 4 were fungicidal.

In contrast, as for the evaluation of the reference products 1 and 2 for biofilm formation inhibitory effect, both of them had a 50% inhibitory concentration above 20 ppm and were poor in the biofilm formation inhibitory effect. As for the fungistatic evaluation, fungal growth was observed in a plot treated with the reference product 1 even at 1000 ppm, and the reference product 1 was not fungistatic. That is, the reference product 1 was not fungicidal. However, no fungal growth was observed in a plot treated with 100 ppm of the reference product 2, and the reference product 2 was fungistatic.

### Production Example 1: Tablet Production

Based on the composition ratio shown in Table 4 below, the ingredients were uniformly mixed, and after addition of water, the mixture was kneaded, dried and fed into a single punch tablet press to give tablets containing the present invention product 1.

**[Table 4]**

| Ingredient | % by mass |
|---|---|
| Glucose | 35.0 |
| Palatinose | 35.0 |
| Gum arabic | 6.5 |
| Flavor | 2.0 |
| Present invention product 1 | 0.5 |
| Water | 21.0 |

### Production Example 2: Film-Shaped Food Product Production

Based on the composition ratio shown in Table 5 below, the ingredients were uniformly mixed, and after addition of water, the mixture was kneaded, spread on a plastic film and dried to give a film-shaped food product containing the present invention product 1.

**[Table 5]**

| Ingredient | % by mass |
|---|---|
| Hydroxypropyl methylcellulose | 70.0 |
| Microcrystalline cellulose | 5.0 |
| Glycerin | 20.5 |
| Sodium saccharin | 0.5 |
| Peppermint flavor | 1.5 |
| d-α-Tocopherol preparation | 1.0 |
| Present invention product 1 | 0.5 |
| Water | 1.0 |

### INDUSTRIAL APPLICABILITY

The present invention provides a biofilm formation inhibitor that can be used in the fields of food industry, cosmetic industry, medical care, etc.

## Claims

1. A biofilm formation inhibitor comprising monoglyceryl dicaprylate and/or diglyceryl dicaprylate as an active ingredient.

2. An oral composition for inhibiting biofilm formation, comprising the biofilm formation inhibitor according to claim 1.

3. A method for inhibiting biofilm formation, comprising using monoglyceryl dicaprylate and/or diglyceryl dicaprylate.
